# EUROPEAN PATENT APPLICATION

(11) **EP 2 204 442 A1**
(43) Date of publication of application: **07.07.2010**
(21) Application number: 08166472.4
(22) Date of filing: 13.10.2008
(51) Int. Cl.: C12N 5/0789

(54) **Method of enriching stem cells in culture**

(30) Priority: 01.10.2008 EP 08165659
(71) Applicant: Fraunhofer-Gesellschaft zur Förderung der angewandten Forschung e.V., 80686 München (DE)
(72) Inventor: Stolzing, Andrea, 04103 Leipzig (DE); Fricke, Stephan, 04229 Leipzig (DE); Miao, Dengshun, Nanjing, Jiangsu 210029 (CN)
(74) Representative: Bühler, Dirk

(57) **Abstract**

The present invention provides a method for enriching stem cells in cell culture comprising the steps of a) culturing a cell mixture comprising stem cells in a suitable cell culture medium in a culture vessel; b) transferring the supernatant comprising non-adherent cells to a new culture vessel in intervals of 6 to 32 hours and c) harvesting the enriched stem cells from the cell culture supernatant. The present invention also provides stem cells enriched by this method, a pharmaceutical composition comprising such stem cells and the use of such stem cells in the preparation of a medicament for the reconstitution of hematopoiesis, for tissue repair and cell therapies.

## Description

The present invention provides a method for enriching stem cells in cell culture comprising the steps of a) culturing a cell mixture comprising stem cells in a suitable cell culture medium in a culture vessel; b) transferring the supernatant comprising non-adherent cells to a new culture vessel in intervals of 6 to 32 hours and c) harvesting the enriched stem cells from the cell culture supernatant. The present invention also provides stem cells enriched by this method, a pharmaceutical composition comprising such stem cells and the use of such stem cells in the preparation of a medicament for the reconstitution of hematopoiesis, for tissue repair and cell therapies.

Adult bone marrow contains both hematopoietic stem cells and mesenchymal stem cells.

The hematopoietic stem cell (HSC) is the progenitor for all blood cells, from which the entire mature hematopoietic system comprising lymphocytes (B and T cells of the immune system) and myeloid cells (erythrocytes, megakaryocytes, granulocytes and macrophages) are formed.

Due to these properties, hematopoietic stem cell transplantation (HSCT) is currently used in clinical applications for the treatment of a variety of diseases ranging from cancer, autoimmunity, aplastic anemia and other hematological diseases (Blazar and Murphy (2005) Philos.Trans.R.Soc.Lond B Biol.Sci. 360: 1747-1767). For example, allogeneic hematopoietic stem cell transplantation (HSCT) is the only curative treatment for many patients with hematological malignancies (Aschan J. (2006) Br. Med. Bull. 77-78: 23-36; Shlomchik (2007) Nat.Rev.Immunol.7(5): 340-352).

Stem cell sources for HSCT are autologous and allogeneic bone marrow, peripheral blood stem cells (PBS) and umbilical cord blood stem cells.

However, when allogeneic bone marrow or hematopoietic stem cells are transplanted, there is the risk that the recipient develops a Graft-versus-Host-Disease which is an immunological reaction against the transplant. This reaction is mainly mediated by T lymphocytes present in the transplant which recognize the human leukocyte antigen on the organs of the recipient as foreign and therefore elicit an immune response which leads to symptoms mainly in skin, liver, colon and eye. To minimize the risk of developing a Graft-versus-Host-Disease immunosuppressive agents such as cyclosporine, glucocorticoids, antimetabolites and monoclonal antilymphocyte antibodies are administered to patients receiving the transplant. However, it is also desirable to eliminate the cells mediating the Graft-versus-Host-Disease in the transplant before transplantation by a simple method which does not require a long purification procedure.

At the same time, the stem cell transplant should be able to restore hematopoiesis efficiently. Therefore there is still a need for the isolation of new stem cell sources.

Mesenchymal stem cells (MSCs) might be an alternative and one of the most promising stem cell sources for hematopoietic stem cell transplantation because they are easily available and modest regarding their requirements for *in vitro* expansion and genetic manipulation (Javazon et al. (2004) Exp.Hematol. 32:414-425; Baron and Gothot (2007) Rev.Med.Liege 62 Spec No:9-14).

Previous studies and case reports on patient have shown that the co-transplantation of mesenchymal stem cells resulted in fast engraftment (Le et al. (2007) Leukemia 21: 1733-1738; Arber et al. (2003) Blood 102: 421-428; Stagg (2007) Tissue Antigens 69: 1-9). In 1974 Friedenstein et al. were the first who described a stem cell population in the bone marrow stroma which is multipotent and has the capacity to differentiate *in vitro* and *in vivo* into different mesenchymal lineages such as bone, cartilage, adipose, tendon and bone marrow stroma (Friedenstein et al. (1974) Exp.Hematol. 2: 83-92).

MSCs play a major role in hematopoiesis as they provide growth factors which are essential for hematopoiesis (Deans and Moseley (2000) Exp.Hematol. 28: 875-884; Zhu et al. (2003) Zhongguo Shi Yan.Xue.Ye.Xue.Za Zhi. 11:115-119). After transplantation into non-irradiated hosts, MSCs were able to generate hematopoietic cells and to repair tissues of liver, lung and kidney (Semont et al. (2006) Adv. Exp. Med Biol. 585: 19-30). MSCs show a high self renewal capacity and multi-lineage potentiality (Baksh et al. (2004) J. Cell Mol. Med. 8: 301-316).

Originally, MSCs were defined as a *in vitro* highly adherent fibroblastic cell fraction which attach within 24 hours (Delorme and Charbord (2007) Methods Mol. Med. 140: 67-81). However, it is not known whether MSCs reside in bone marrow as adherent fibroblastic or non-adherent round cells. These non-adherent cells could serve as a common stem cell for both hematopoietic and stromal lineages and a major source for adult stem cells.

Adult stem cells are used in many cell based therapies and for tissue engineering. The isolation of adult stem cells, the expansion and culturing of adult stem cells is highly important for the therapeutic success of cell based therapies. In the moment the use of highly homogenous stem cell populations is favored, however the interaction of stem cell subpopulations and the use of the earliest forms of stem cells is desirable to improve the therapy success.

Homogenous cell compositions are commonly expanded in bioreactors. However, such cell culture systems often aim at expanding a single cell type such as hematopoietic stem cells, thereby ignoring beneficial interactions between different cell types. In some cases, MSC are only added to HSC during transplantations after they have been cultivated separately (US 5,733,542). However, this approach requires the use of two different preparations and is therefore more complex. Furthermore, bioreactor cultures require specialized equipment and cannot be used in a standard cell culture laboratory.

The enrichment of stem cells after culturing a mixture of the stem cells with other cells is usually accomplished by depletion of undesired cells by magnetic beads or cell sorting. This however requires additional equipment and increases the risk of contaminating the cell preparation. Furthermore, it may change the cell behaviour.

Therefore, there is still a great need for a simple cell culture process which leads to a depletion of T cells and which at the same time allows for the enrichment of stem cells which support the reconstitution of hematopoiesis and/or organ repair in a recipient.

### OBJECT AND SUMMARY OF THE INVENTION

It is an object of the present invention to provide a cell culture process which is effective in depleting T cells and promoting the simultaneous enrichment of stem cells.

It is a further object of the present invention to provide a stem cell population which may efficiently be used in the reconstitution of hematopoiesis as well as in the treatment of Alzheimer's disease and other degenerative diseases, ageing, bone healing, myocardial infarction and others.

These and further objects of the invention, as will become apparent from the description, are attained by the subject-matter of the independent claims.

Further embodiments of the invention are defined by the dependent claims.

The inventors of the present invention have surprisingly found that stem cells may be enriched by a simple method which does not involve the use of complex media or the introduction of purification steps after culturing the cells. The culture system improves the *in vitro* expansion of the cells and minimizes in *vitro* ageing. For the treatment of humans it is particularly advantageous that the cells may be used after only a short expansion period. Furthermore, there is only little influence on the cells, as the *in vivo* environment is reproduced *in vitro* by use of the entire adult stem cell population without isolating a particular stem cell type such as hematopoietic stem cells and mesenchymal stem cells. Thus, the stem cells of the present invention possess great therapeutic potential in regenerative medicine.

The stem cells enriched by the method of the invention show increased reconstitution of hematopoiesis compared to bone marrow transplantation and mesenchymal stem cell transplantation. This means that the transplantation of a lower amount of these cells compared to bone marrow cells is sufficient to protect mice from radiation-induced death and that hematopoiesis is recovered earlier than with the transplantation of bone marrow. Furthermore, these cells show increased migratory activities *in vitro.* The age of the donor seems to be less important and the cells show lower ageing marker expression.

According to one aspect of the invention a method of enriching stem cells in cell culture is provided, comprising the steps of:
a) culturing a cell mixture comprising stem cells in a suitable cell culture medium in a culture vessel,
b) transferring the supernatant comprising non-adherent cells to a new culture vessel in intervals of 6 to 32 hours; and
c) harvesting the enriched stem cells from the cell culture supernatant.

In a preferred embodiment of the present invention the supernatant is transferred to a new culture vessel at least three times.

In a further preferred embodiment of the present invention the cell culture medium is not exchanged during the transfer of the cells.

In a further embodiment of the present invention, the culture vessel is treated for tissue culture.

In still another embodiment of the present invention the cell mixture comprising the stem cells is seeded in a density of 10⁵ to 10⁷ cells per ml in the cell culture medium before culturing the cell mixture comprising the stem cells.

In one embodiment of the present invention the cell mixture comprising the stem cells is obtained from a suitable stem cell source, preferably from bone marrow.

In a further embodiment, the stem cells are of mammalian origin, preferably of human origin.

In one embodiment of the present invention the enriched stem cells are positive for at least one marker selected from the group consisting of CD11b, CD31, CD44, CD45, CD90, CD105 and CD133.

In a further aspect of the present invention, stem cells enriched by the method of the present invention are provided. In still a further aspect of the present invention a pharmaceutical composition comprising such stem cells is provided.

The present invention is also directed to the use of the stem cells prepared by the method of the present invention as a medicament, preferably for the reconstitution of hematopoiesis, the treatment of Alzheimer's disease and/or ageing and for bone healing.

In still a further aspect of the present invention an isolated cell population which is positive for the markers CD11b, CD31, CD44, CD45, CD90, CD105 and CD133 is provided and its use as a medicament, for example for the reconstitution of hematopoiesis, the treatment of Alzheimer's disease and/or ageing and for bone healing are provided.

### DESCRIPTION OF THE DRAWINGS

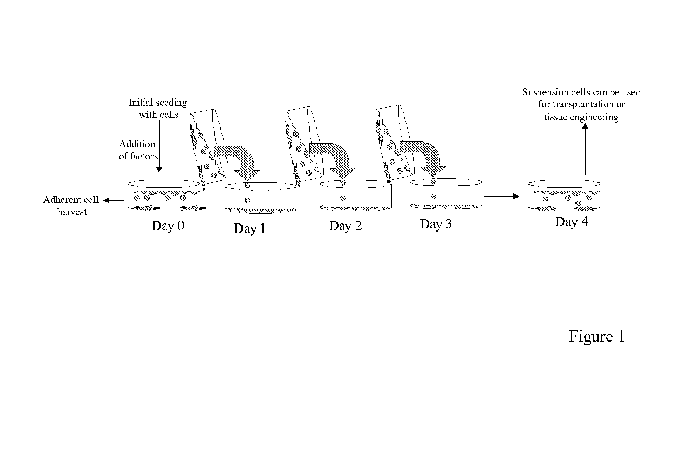

Figure 1: Schematic drawing of the cell culture process of the present invention, wherein the cells are transferred to a new vessel in intervals of 24 hours and harvested after four days of culture.
Figure 2: Flow cytometric analysis of the cells enriched by the cell culture process of the present invention.
   a) Flow cytometric analysis of the supernatant of the cell cultures for CD45 expression on the different days of cultivation using a fluorescence-labelled antibody.
   b) Flow cytometric analysis of the supernatant of the cell cultures for CD31 expression on the different days of cultivation using a fluorescence-labelled antibody.
   c) Flow cytometric analysis of the supernatant of the cell cultures for CD44 expression on the different days of cultivation using a fluorescence-labelled antibody.
   d) Flow cytometric analysis of the supernatant of the cell cultures for CD11b expression on the different days of cultivation using a fluorescence-labelled antibody.
   e) Flow cytometric analysis of the supernatant of the cell cultures for CD90 expression on the different days of cultivation using a fluorescence-labelled antibody.
   f) Flow cytometric analysis of the supernatant of the cell cultures for CD105 expression on the different days of cultivation using a fluorescence-labelled antibody.
   g) Flow cytometric analysis of the supernatant of the cell cultures for CD133 expression on the different days of cultivation using a fluorescence-labelled antibody.
   h) Flow cytometric analysis of the supernatant of the cell cultures for CD45 and B220 expression on the different days of cultivation using a fluorescence-labelled antibody.
   i) Flow cytometric analysis of the supernatant of the cell cultures for F4/80 expression on the different days of cultivation using a fluorescence-labelled antibody.
   j) Quantification of the results obtained by flow cytometric analysis with cells derived from Balb/C mice after four days of culture.
   k) Quantification of the results obtained by flow cytometric analysis with cells derived from C57B1/6 mice after four days of culture.

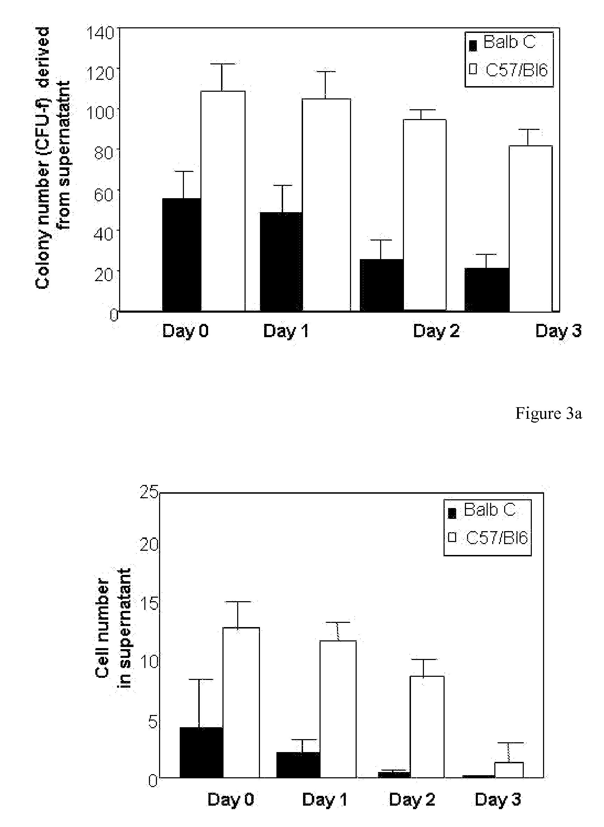

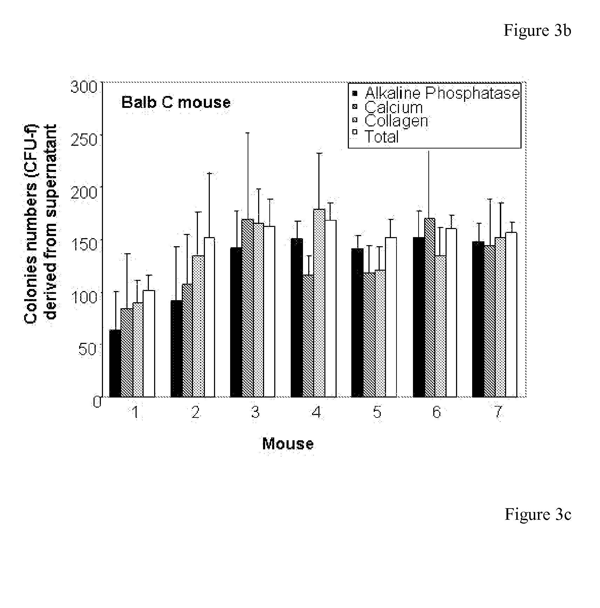

Figure 3: Differentiation potential to mesenchymal cells of the cells cultured according to the process of the present invention
   a) Number of colonies (CFU-f) derived from the supernatant of the cell culture on different days of culture of bone marrow cells from both Balb C and C57/B16 mice.
   b) Cell number in the supernatant of the cell culture on different days of culture of bone marrow cells from both Balb C and C57/B16 mice.
   c) Number of colonies (CFU-f) derived from the supernatant of the cell culture on day 4 of the culture of bone marrow cells from seven different Balb C mice which stained positive for alkaline phosphatase, calcium and collagen and total number of CFU-fs.

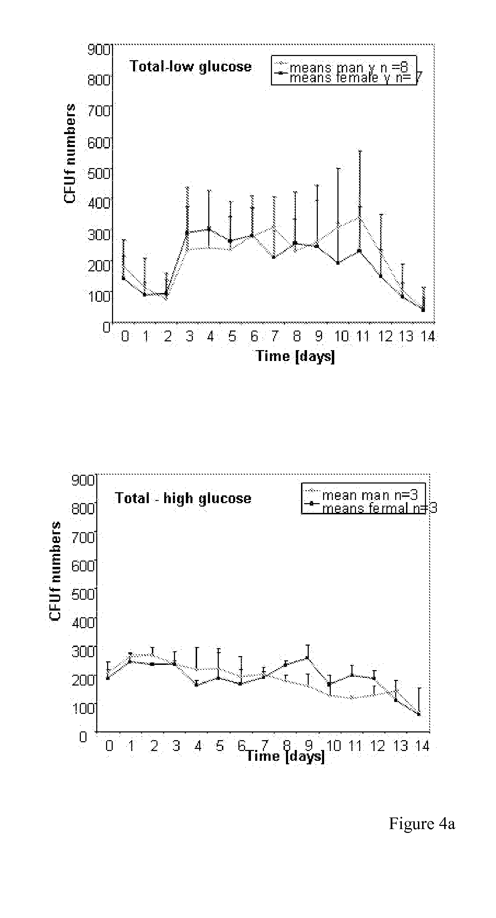

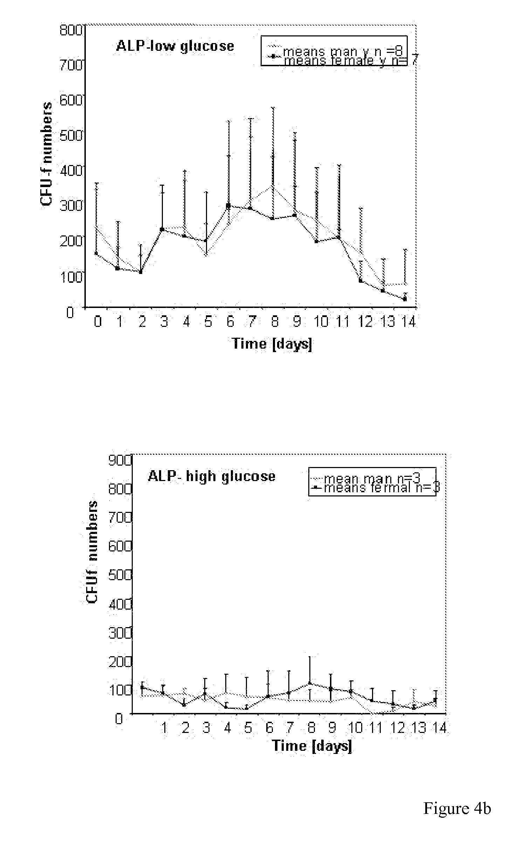

Figure 4: Influence of the amount of glucose in the cell culture medium on the number of mesenchymal stem cells derivable from the supernatant of the cell culture of bone marrow from Sprague Dawley rats.
   a) Total CFU-f numbers derived from bone marrow cultures grown in DMEM medium with low (1 g/l) or high (4.5 g/l) glucose content on different days of CFU-f culture.
   b) CFU-f numbers derived from bone marrow cultures grown in DMEM medium with low (1 g/l) or high (4.5 g/l) glucose content staining positive for alkaline phosphatase on different days of CFU-f culture.

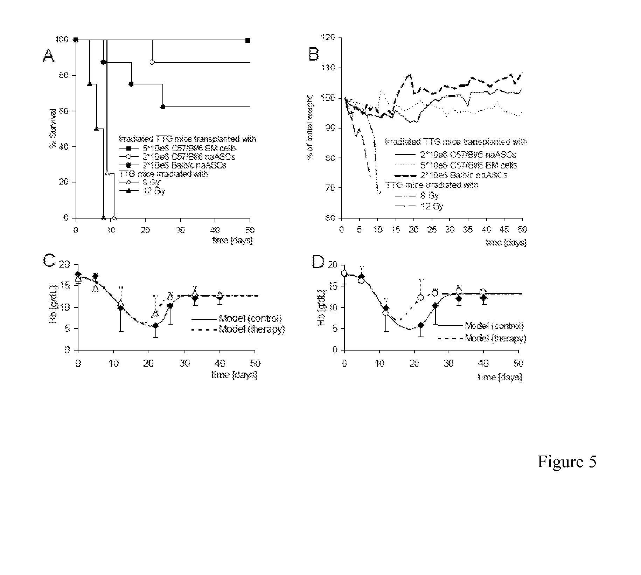

Figure 5: Survival analysis (A) and body weight (B) and recovery models (C-D) after transplantation and irradiation
   Transgenic mice (C57/B1/6) received either syngeneic naASCs from C57/B1/6 mice (2 x 10⁶ cells), syngeneic bone marrow from C57/B1/6 mice (5 x 10⁶ cells), 2 x 10⁶ allogeneic naASCs from Balb/c mice or 0.9% NaCl. The body weight of the mice surviving the irradiation was determined over a period of 50 days. Recovery rate of Hb (model therapy) after lethal irradiation and transplantation of allogeneic naASCs (C) and syngeneic naASCs (D) compared to syngeneic bone marrow transplantation (model control).

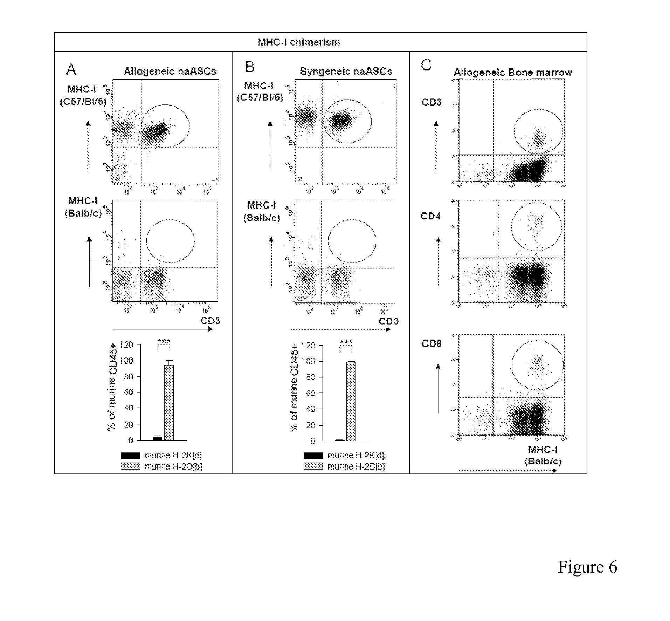

Figure 6: Flow cytometric analysis of MHC-I and CD3 on day -2 before and day 40 after transplantation.
   MHC-I (H-2D[b], H-2D[d]) and CD3 levels from peripheral blood (gated for lymphocytes) on day 40 after transplantation of allogeneic naASCs (A), syngeneic naASCs (B) or allogeneic bone marrow (C). In animals transplanted with allogeneic bone marrow additionally murine CD4/H-2K[d] and CD8/H-2K[d] expression on lymphocytes was analyzed.

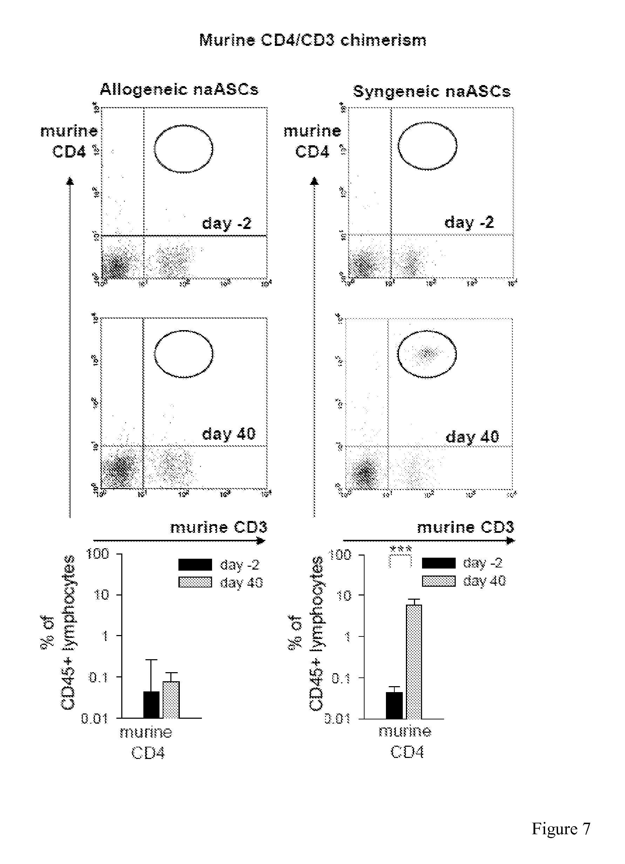

Figure 7: Flow cytometry analysis of murine CD4/CD3 (gated for lymphocytes) on day -2 before and day 40 after transplantation
   Data are shown for transplantation of allogeneic and syngeneic naASCs.

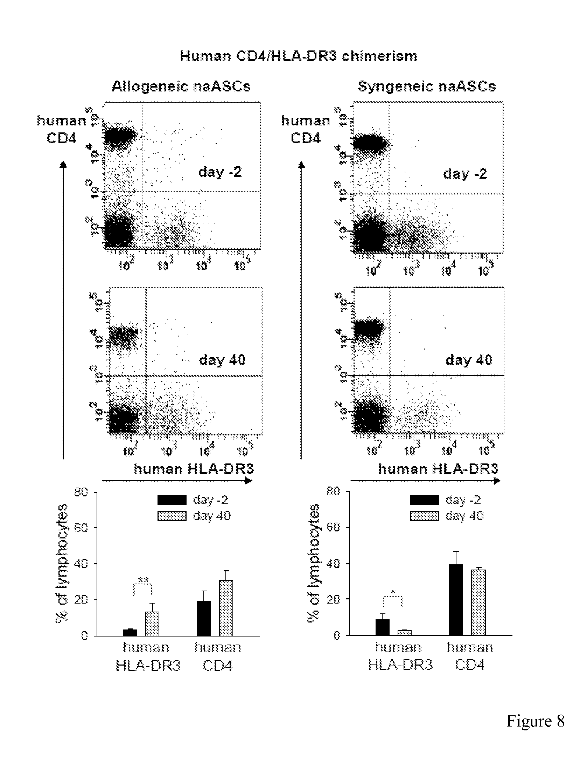

Figure 8: Flow cytometry analysis of human CD4/HLA-DR3 (gated for lymphocytes) from peripheral blood on day -2 before and day 40 after transplantation
   Data are shown for transplantation of allogeneic and syngeneic naASCs.

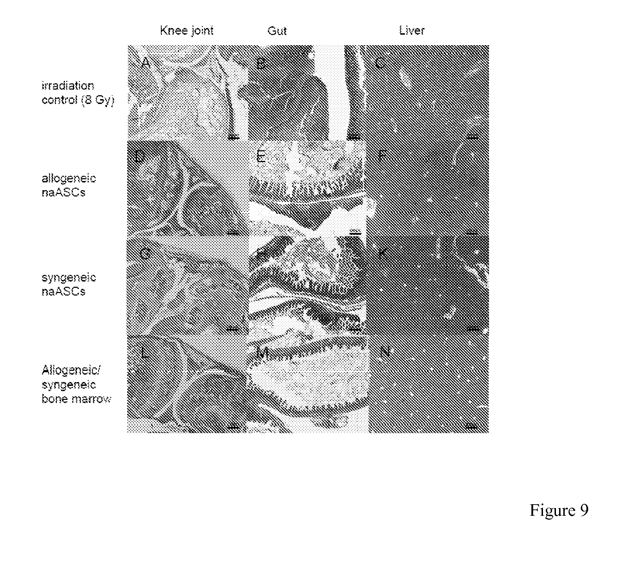

Figure 9: Kaolin aniline orange G (KAO) staining of knee joints and hematoxylin eosin (HE) staining of gut and liver from control (staining day 8-12) and transplanted triple transgenic mice (staining day 50)
   Kaolin aniline orange G (KAO) staining of knee joints and hematoxylin eosin (HE) staining of gut and liver from control (staining day 8-12) and transplanted triple transgenic mice (staining day 50). Lethally irradiated mice (A-C), mice transplanted with allogeneic naASCs (D-F), mice transplanted with syngeneic naASCs (G-K), and mice transplanted with allogeneic bone marrow (L-N). Shown at x 20 original magnification.

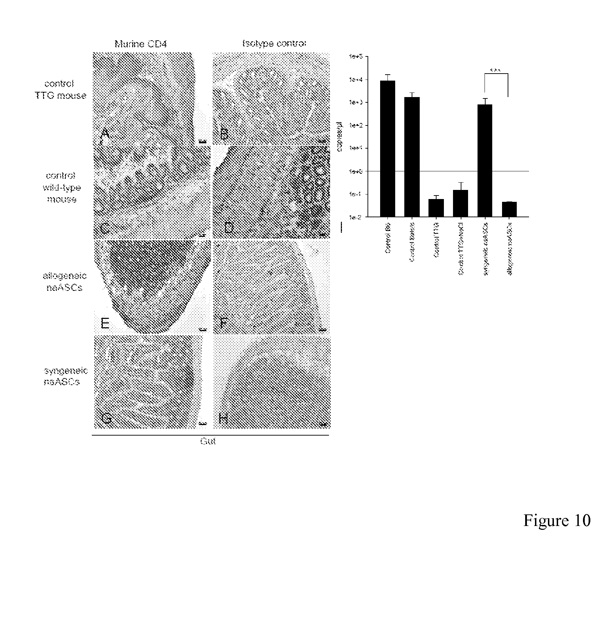

Figure 10: Murine CD4 and isotype control immunohistology staining (streptavidine peroxidase technique) and real-time PCR of gut (day 50)
   Murine CD4 and isotype control immunohistology staining (streptavidine peroxidase technique) and real-time PCR of gut (day 50). Triple transgenic mice (A-B), C57/B1/6 wild-type mice (C-D), mice transplanted with allogeneic naASCs (E-F), mice transplanted with syngeneic naASCs (G-H). Shown at x 20 original magnification. Quantification of murine CD4 by real-time PCR (I).

### DETAILED DESCRIPTION OF EXEMPLARY EMBODIMENTS

Before describing in detail exemplary embodiments of the present invention, the following definitions are given.

The term "approximately" describes a deviation of the indicated value by at least 10 %, preferably by at least 5 % and most preferably by at least 1 %.

The term "stem cells" refers to cells which have retained the capacity to proliferate and differentiate into different cell types. Stem cells enriched in accordance with the present invention are pluripotent stem cells, i.e. stem cells which have retained the capacity to differentiate into distinct cell lineages and cell types.

It is understood that the term "stem cells" in accordance with the invention does not comprise human embryos. Furthermore, it is understood that the term "stem cells" does not comprise pluripotent stem cells which have been directly derived from a human embryo. Embryonic stem cells which have been derived from publicly available and previously established stem cell lines are understood to fall within the meaning of the term "stem cells" as used by the present invention.

In a preferred embodiment, the stem cells enriched by the method of the present invention include stem cells of the hematopoietic and mesenchymal lineage. The term "stem cells of the hematopoietic and mesenchymal lineage" is intended to mean that the cells enriched by the method of the present invention are capable of differentiating to hematopoietic cells and mesenchymal cells. However, this does not necessarily mean that these cells meet with the classical definition of hematopoietic and mesenchymal stem cells. For example, mesenchymal stem cells are commonly identified and isolated by their ability to adhere to the plastic surface of the culture vessel. The stem cells isolated by the method of the present invention do not adhere to the surface, but nevertheless show other characteristics of mesenchymal stem cells such as the expression of alkaline phosphatase and collagen.

The stem cells enriched by the method of the present invention may also be called naASCs (non-adherent adult stem cells).

Besides culturing native, i.e. not genetically altered, stem cells, the cell culture process of the present invention may also be used to enrich genetically altered cells. Several strategies have been employed to deliver transgenes into stem cells, most of them using viral vectors such as oncogenic retroviruses, lentivirus-based vectors, adenoviral vectors and adeno-associated viruses. Non-viral methods for transgene delivery include electric field-induced molecular vibration, electroporation, magentofection and liposome-based transfection.

After the stem cells are obtained from a suitable stem cell source, they are present in a cell mixture with other cell types such as T cells and macrophages. During the cell culture process of the present invention these other cell types are depleted from the cell mixture, while the stem cells are enriched.

The other cell types are depleted by at least 20% or 30%, preferably at least 40% or 50%, more preferably at least 60% or 70%, even more preferably at least 80% or 90% and most preferably by at least 95% or 98%.

The term "syngeneic stem cells" means that the stem cells that are transplanted into an individual are derived from a genetically identical twin of the individual. For the transplantation of murine stem cells to another mouse this term means that the stem cells that are transplanted are derived from the same mouse strain as the recipient and from the same litter.

In contrast, the term "allogeneic stem cells" means that the stem cells that are transplanted into an individual are derived from an individual which is not genetically identical to the recipient. For the transplantation of murine stem cells this term means that the stem cells that are transplanted are derived from a mouse strain that is not the same as the recipient mouse strain.

The term "enriching" means that the percentage of the stem cells relative to the total number of cells in a given volume of cell culture medium increases over the time of culture of the cells. At the same time this means that the number of other cells present in the cell mixture decreases over the time of culture of the cells. These other cells which are depleted from the cell culture may for example include macrophages and T cells. The cell culture process of the present invention leads to an enrichment of stem cells to at least 10% or 20%, preferably to least 30% or 40%, more preferably to least 50% or 60%, even more preferably to least 70%, 80% or 90% and most preferably to at least 95% or 98% of the total cells in a given volume of cell culture medium.

The terms "cell culture" and "culturing of cells" refer to the maintenance and propagation of cells and preferably animal (including human-derived) cells *in vitro.* Ideally the cultured cells do not differentiate and do not form organized tissues, but undergo mitosis.

"Cell culture medium" is used for the maintenance of cells in culture *in vitro.* For some cell types, the medium may also be sufficient to support the proliferation of the cells in culture. A medium according to the present invention provides nutrients such as energy sources, amino acids and anorganic ions. Additionally, it may contain a dye like phenol red, sodium pyruvate, several vitamins, free fatty acids, antibiotics, anti-oxidants and trace elements.

The term "maintenance of cells" is intended to mean that the cell number of a desired cell population remains substantially unchanged, i.e. neither increases nor decreases.

The term "proliferation of cells" is intended to mean the multiplication of cells thereby leading to an increase in the cell number. The proliferation of cells may be detected by any suitable method. The easiest way to measure proliferation is to seed the cells in a specific, predetermined density and to count the cell number at different time points after seeding. Another way of measuring the proliferation of cells is a [³H]-thymidine incorporation assay which involves the addition of [³H]-thymidine to the cells, incubating them for a specific time, lysing the cells and counting the incorporation of [³H]-thymidine in a scintillation counter. Commercially available kits like the tetrazolium assay (MTT, Sigma) may also be used for measuring proliferation.

For culturing the stem cells according to the present invention any standard medium such as Iscove's Modified Dulbecco's Media (IMDM), alpha-MEM, Dulbecco's Modified Eagle Media (DMEM), RPMI Media and McCoy's Medium is suitable. These media are well-known to a person skilled in the art and may be purchased from companies such as Cambrex, Invitrogen and Sigma-Aldrich. Preferably, the medium is DMEM.

The medium may further comprise a serum component such as horse serum, human serum or fetal calf serum (FCS). Preferably, the medium contains FCS. If present, the serum is present in a concentration of 1-20%, preferably of 3-18%, more preferably of 5-15%, even more preferably of 8-12% and most preferably of 10%.Alternatively the serum component may be replaced by any of several standard serum replacement mixtures which typically include insulin, albumin and lecithin or cholesterol.

In a preferred embodiment, the medium further comprises a corticosteroid such as hydrocortisone, cortisone, dexamethasone and solumedrol. More preferably, the medium comprises dexamethasone. The corticosteroid may be present in a concentration of 10⁻⁶ to 10⁻⁹ M, preferably the concentration is 10⁻⁸ M. Most preferably, the medium comprises dexamethason in a concentration of 10⁻⁸ M.

The medium may additionally comprise one or more additives selected from the group consisting of vitamin D3 (1,25-dihydroxyvitamin D3, cacitriol), resveratrol *(trans-3,* 4', 5-trihydroxystilbene), reversine (2-(4-morpholinoanilino)-11'-cyclohexyladenine), vitamin E (RRR-α-tocopherol), valproic acid (dekapene, valproate, valrelease), EGCG (epigallocatechin-3-gallat) and selenium. Preferably, two of the afore-mentioned additives are present, more preferably three of the afore-mentioned additives are present, even more preferably four of the afore-mentioned additives are present, particularly preferably five of the afore-mentioned additives are present and most preferably all of the afore-mentioned additives are present.

If present, vitamin D3 is present in a concentration from 10⁻⁶ M to 10⁻⁹ M, preferably 10⁻⁸ M. If present, resveratrol is present in a concentration from 10 nM to 5 mM, preferably 0.05 mM. If present, selenium is present in a concentration from 0.1 µM to 10 µM, preferably 0.1 µM. If present, valproic acid is present in a concentration of 0.6 µM to 60 µM, preferably 5 µM. If present, vitamin E is present in a concentration from 1 µM to 10 µM, preferably 10 µM. If present, EGCG is present in a concentration of 0.01 µM. If present, reversine is present in a concentration of 1 µM.

Most preferably, the medium contains vitamin D3 in a concentration of 10⁻⁸ M, resveratrol in a concentration of 0.05 mM, selenium in a concentration of 0.1 µM, valproic acid in a concentration of 5 µM, vitamin E in a concentration of 10 µM, EGCG in a concentration of 0.01 µM and reversine in a concentration of 1 µM.

Furthermore, the medium may comprise vitamin C in the form of ascorbic acid or 2-phosphate ascorbic acid. If present, the concentration of vitamin C is 50 µg/ml.

Most preferably, the medium is DMEM containing 10⁻⁸ M dexamethasone, 10% FCS, 50 µg/ml vitamin C, 10⁻⁸ M vitamin D3, 0.05 mM resveratrol, 0.1 µM selenium, 5 µM valproic acid, 10 µM vitamin E, 0.01 µM EGCG and 1 µM reversine.

The glucose content of the medium is below 4.6 g/l, preferably below 4.5 g/l, more preferably below 4 g/l, even more preferably below 3 g/l, particularly preferably below 2 g/l and most preferably it is 1 g/l. DMEM media containing 1 g/l glucose are commercially available as "DMEM low glucose" from companies such as PAA, Omega Scientific, Perbio and Biosera.

The culture is kept at oxygen below 21 % and temperature below 38°C.

A "culture vessel" is any vessel which is suitable for growing cells in a culture medium or agar, either in fluid phase or adhered to an interior surface of the container. Types of such specialized vessels include roller bottles, spinner flasks, petri dishes and tissue flasks. Culture vessels are designed to be incubated in temperature, humidity and gas controlled environments to facilitate maximum cell or tissue growth. Generally, a layer of cell culture medium or agar covers the growing surface. The portion of the vessel not utilized as a growing surface encloses the interior gaseous environment which surrounds the cell culture. Cells, tissues, microorganisms and the like typically are introduced into the interior of cell culture vessels through an opening in the vessel. After introduction of the cells the opening may be closed such that the cells are not in contact with the environment during the culturing of the cells.

In a preferred embodiment of the present invention, the culture vessel is treated for tissue culture, which means that the surface of the culture vessel is treated such that cells which usually grow in an adherent state grow adherently, but cells which grow in suspension do not adhere or adhere only loosely. Such treatment may involve the irradiation of the vessel or the coating of the vessel, for example with a special plastic, polymer or nanostructure or with proteins of the extracellular matrix. Such vessels can comprise tissue culture dishes and tissue culture flasks and are available from different suppliers such as Becton Dickinson, Greiner, Sigma and TPP.

The term "transferring the cells" means that the supernatant, i.e. the cell culture medium containing the stem cells of the present invention which medium covers the layer of cells growing in an adherent state, is removed from the culture vessel and seeded into a new vessel, i.e. a vessel which was not in contact with cells before the supernatant is transferred to it. The supernatant may for example be removed from the culture vessel by means of a pipette or it may be poured off. To transfer also cells which loosely adhere to the surface of the culture vessel, the surface may be washed with a pipette using the medium present in the culture vessel. The step of transferring the cells may involve additional steps, such as the step of centrifuging the supernatant and taking up the cells in the medium already used in the previous vessel or partially or completely in fresh medium after removing part or all of the used medium.

Preferably, the cells are transferred directly, without centrifugation, from one vessel to the other.

Also preferably, the cell culture medium is not exchanged when the cells are transferred, i.e. the same medium as used in the old vessel is also used in the new vessel. Fresh medium is only added to adjust the volume of the culture medium to a predetermined volume so as to balance the loss of medium which may occur during the transfer of the cells. For example, if the cells are cultured in a volume of 10 ml culture medium and the volume of medium transferred to the new vessel is only 9.5 ml, 0.5 ml of fresh medium are added. This addition of fresh medium for volume adjustment is also intended to be comprised within the term "the medium is not exchanged during transfer of the cells". A continuous and slow medium exchange system would also be possible.

The term "non-adherent cells" is intended to comprise both cells which are present in the supernatant of the cell culture and cells which are loosely attached to the surface of the vessel, but can be removed by flushing the surface with medium one or more times.

The non-adherent cells are transferred to a new vessel in intervals of 6 to 32 hours, preferably 10 to 28 hours, more preferably 15 to 26 hours and most preferably 24 hours.

In one embodiment of the present invention the supernatant is transferred to a new vessel at least one or two times, preferably three times, four times or five times before the stem cells of the present invention are harvested. Most preferably, the supernatant is transferred at least three times. However, it is also possible to transfer the supernatant more often, such as ten times, twenty times or thirty times.

The term "seeding the cells" generally refers to introducing a cell mixture into an *in vitro* environment capable of promoting the survival and/or growth of the introduced cells or at least a certain population within the cell mixture such as the stem cells in the present case. The cells seeded in the method of the present invention are the cells obtained from a suitable stem cell source such as bone marrow and comprise a mixture of stem cells with other cell types such as T cells and macrophages. In a preferred embodiment the cells are seeded immediately after their isolation. However, it is also possible to introduce one or more additional steps between the isolation and the seeding, such as freezing or centrifuging the cells.

The term "isolation of cells" is intended to mean the step of obtaining the cell mixture from a suitable stem cell source. In the case of isolation from the bone marrow, the bone may be flushed with an appropriate solution such as medium or an acceptable buffer such as Hepes, phosphate buffers and lactate buffers and the cells are collected. Alternatively and preferably, the cells are isolated from the bone marrow by centrifugation according to the method described in Dobson et al. (1999) Calcif. Tissue Int. 65(5): 411-413.

The cells are seeded in a density of 10⁵ to 10⁷ cells per ml of cell culture medium, preferably in a density of 2 x 10⁵ to 5 x 10⁶ per ml of cell culture medium, more preferably in a density of 3 x 10⁵ to 1 x 10⁶ per ml of cell culture medium and most preferably in a density of 4 x 10⁵ per ml of cell culture medium.

A "suitable stem cell source" is a tissue within the human or animal body which comprises the stem cells of the present invention together with other cell types. Preferably, the suitable stem cell source is bone marrow, both adult and fetal, cytokine or chemotherapy mobilized peripheral blood, fetal liver, umbilical cord blood, embryonic yolk sac and spleen, both adult and fetal, more preferably the stem cell source is adult bone marrow or umbilical cord blood, most preferably the stem cell source is bone marrow. Bone marrow cells may be obtained from any known source, including, but not limited to, ilium, sternu, tibiae, femora, spine or other bone cavities.

Preferably, the stem cells are isolated from a mammalian organism such as human, mouse or rat, and more preferably the stem cells are isolated from a human organism.

The stem cells enriched by the method of the present invention may be harvested for further use. The term "harvesting the cells" is intended to mean that the cells are withdrawn from the cell culture and optionally processed for further use. The cells may be harvested by obtaining the supernatant containing the non-adherent cells. The cells may be harvested after the supernatant containing the cells has been transferred at least three times, at least four times, at least five times or at least six times into a new vessel. The cells may then be centrifuged to remove the cell culture medium. Furthermore, the cells may be washed one or more times, for example in PBS (phosphate buffered saline) or other buffered saline solutions. The term "harvesting the cells" is not intended to comprise a further seeding and/or cultivating the cells. In particular, it does not comprise the culturing of the harvested cells from the supernatant in an adherent state. Furthermore, it does not comprise the culturing of the harvested cells from the supernatant to enrich for mesenchymal stem cells.

The harvested cells may be used directly for transplantation into a mammal or may be cryoconserved by freezing them at a temperature from about - 196 °C to about - 140°C.

The enriched stem cells may be characterized by the expression of certain surface markers. These surface markers are usually given a cluster of differentiation (CD) designation which describes groups of immunophenotypical surface characteristics of cells. Usually CD molecules are membrane-bound glycoproteins which are recognized by a cluster of monoclonal antibodies that display the same cellular reactivity. These surface molecules may for example be detected by means of a flow cytometer such as a fluorescence-activated cell sorter (FACS) manufactured by companies such as Becton Dickinson.

The method of the present invention leads to an enrichment of cells which are positive for at least one marker selected from the group consisting of CD44, CD31, CD11b, CD45, CD90, CD105 and CD133. Particularly, the method of the present invention enriches stem cells which are positive for CD11b. Preferably, the method of the present invention enriches a cell population which is positive for the markers CD44, CD31, CD11b, CD45, CD90, CD105 and CD133. Furthermore, the method of the present invention leads to a depletion of CD4, CD8, CD19 and CD117 positive cells within the CD45 positive subfraction. The method of the present invention also leads to an enrichment of cells which are positive for the markers CD45/B220 and F4/80 which together with the fact that the cells are also positive for CD11b indicates that the stem cells enriched by the method of the present invention are capable of differentiating to microglia cells.

The pharmaceutical composition of the present invention comprises the enriched stem cells, optionally together with a pharmaceutically acceptable carrier such as saline, a pH balanced buffer, such as a phosphate, citrate or bicarbonate buffer in a saline solution, albumin or dextrose. Optionally, the pharmaceutical composition may comprise another active ingredient such as a growth factor which stimulates the proliferation of cells. The pharmaceutical composition is formulated in accordance with routine procedures for administration to mammals.

For transplantation into humans the amount of stem cells within the pharmaceutical composition typically is between 1 x 10⁶ and 1 x 10⁷ cells per kg body weight, preferably between 2 x 10⁶ and 4 x 10⁶ cells per kg body weight. For autologous transplantation, i.e. the transplantation of stem cells which are derived from the same person that receives the transplant, usually 2 x 10⁶ cells per kg body weight are sufficient. In allogeneic transplantations, i.e. the transplantation of stem cells from another person, 3 to 4 x 10⁶ cells may be transplanted. It is noted that the stem cells enriched by the method of the present invention are more effective in reconstituting hematopoiesis than bone marrow cells. Hence, the same amount of cells may lead to a more rapid reconstitution of hematopoiesis.

Two or more transplantations of the stem cells of the present invention may be necessary to fully reconstitute hematopoiesis.

The stem cells enriched by the method of the present invention may be administered by systemic intravenous injection. They may be administered by any convenient route, for example by infusion or bolus injection.

The stem cells enriched by the method of the present invention may be used in the reconstitution of hematopoiesis in individuals in which hematopoiesis is disturbed and fewer blood cells may be detected, preferably due to chemotherapy and/or radiation therapy or an accident which involves exposure to radioactive irradiation. For example, chemotherapy often results in myelosuppression and myeloablation. When stem cells are transplanted, they may replace the functional hematopoietic system or may trigger residual stem cells within the recipient to differentiate to blood cells. The reconstitution of hematopoiesis may be monitored by flow cytometry before and after stem cell transplantation. By flow cytometry the presence of hematopoietic cells may be detected. For example, B cells may be detected using an antibody against CD 19 and T cells may be detected using an antibody against CD4 or CD8. Another way to monitor the reconstitution of hematopoiesis is the determination of the hemoglobin content of the blood which indicates the presence and amount of erythrocytes.

Due to their ability to differentiate to mesenchymal cells, the stem cells enriched by the method of the present invention may further be used in bone healing and regeneration or spine surgery. Further they may be used to treat cartilage defects, ligament and tendon injuries and other musculoskeletal and connective tissue disorders, osteoarthritis or rheumatoid arthritis and autoimmune reactions such as collagenopathies and multiple sclerosis. Furthermore, the stem cells may be used to treat Alzheimer's disease and other degenerative diseases, ageing, myocardial infarction and others

As the stem cells enriched by the method of the present invention also trigger stem cells of the host into which the stem cells are transplanted, they may also trigger aged or otherwise dormant stem cells and induce *in vivo* regeneration. This might be dangerous in cancer patients as it might trigger tumor or leukemia stem cells and consequently lead to a higher incidence of tumor relapses. Hence, it may also be desirable to deplete these cells from a bone marrow transplant to minimize the risk of tumor relapse. One way to deplete the cells from bone marrow is by the use of magnetic beads on which a specific antibody recognizing a surface molecule is immobilized. The bone marrow sample is then added to the magnetic beads and the cells which become attached to the magnetic beads due to antibody binding can be separated magnetically. Another way to separate the stem cells isolated by the method of the present invention from a bone marrow sample is by means of fluorescence-activated cell sorting, wherein the cells are incubated with an antibody to the antigen of interest which antibody has been conjugated to a fluorescent dye. The fluorescent dye is then excited by a laser beam to emit light which is detected by a photo-multiplier tube which is specific for the emission wavelength of the fluorochrome by virtue of a set of optical filters and the cells may be separated. Furthermore, the cells could be depleted by using an antibody coupled to a cytotoxic agent such as complement or cytotoxins.

The stem cells of the present invention could be depleted by virtue of their expression of CD90, CD105 and CD133, i.e. antibodies against one or more of these surface markers could be used to separate the cells from the other cells present in the cell mixture.

The term "isolated cell population" is intended to mean that the cells are not in contact with other cells with which they are usually in contact within the body of the mammal or in a tissue sample obtained directly, i.e. without purification or enrichment step, from the mammal. A "cell population" according to the present invention may comprise not only cells of one cell type such as hematopoietic stem cells or mesenchymal stem cells as defined for example by the expression of a specific combination of surface markers, but also a mixture of cells of different cell types which show different combinations of surface markers.

The invention is further illustrated by the following examples, which should not be construed as limiting. The contents of all references, patent applications, patents, published patent applications, tables and appendices cited throughout this application are hereby incorporated by reference.

### EXAMPLES

### 1. Animals

C57/B1/6 CD4^{k/o} mice transgenic for human CD4 and HLA-DR (TTG mice; Laub et al. (2000) J Immunol Methods 246: 37-50) were bred at the Animal Facility at the University of Leipzig. The mice have a completely functional murine immune system which is only modified regarding CD4, and additional HLA-DR.

The modified molecules allow a unique analysis of chimerism and the discrimination between donor and host hematopoiesis even in a syngeneic transplantation setting. Because the murine CD4 molecule is knocked out before transplantation, murine stem cell engraftment can be characterized by arising T cells expressing murine CD4. Human CD4 and HLA-DR3 represent the autologous hematopoiesis.

The mice strain was maintained under standardized conditions. The control environment was characterized by a temperature of 20-22°C, 50% humidity and an artificial 12 h light/dark cycle and the mice were kept under these conditions already two weeks before starting the experiment. The animals were kept on soft-wood bedding in Macrolon^{®} type II and III cages (Ehret, Emmendingen, Germany). The mice had free access to autoclaved food (22% protein, 4.5% raw fibre, 7% ash, utilizing energy 3.1 kcal/g (Altromin 1324, Lage, Germany) and water. As donors, C57/B1/6 and Balb/C mice were purchased from Charles River (Sulzfeld, Germany; http://jaxmice.jax.org). All mice were housed, treated and handled in accordance with the guidelines set forth by the University of Leipzig Animal Care Committee and the Regional Board of Animal Care for Leipzig (animal experiment number 24/06).

### 2. Method for enriching stem cells in cell culture

2x10⁶ BMCs derived from C57/B1/6 and Balb/C wild-type mice, respectively, were cultured in tissue culture plates in 5ml of Dulbecco's modified Eagle's minimal essential medium (DMEM) containing 10% fetal calf serum (FCS, Invitrogen) and 10⁻⁸ M dexamethasone. The non-adherent cells in the supernatants from the total BMCs were transferred into a new dish every day for 4 days and then harvested by obtaining the supernatant, pelleting the cells and washing them three times with normal saline (0.9% NaCl).

### 3. Characterization of the cultured cells by flow cytometry

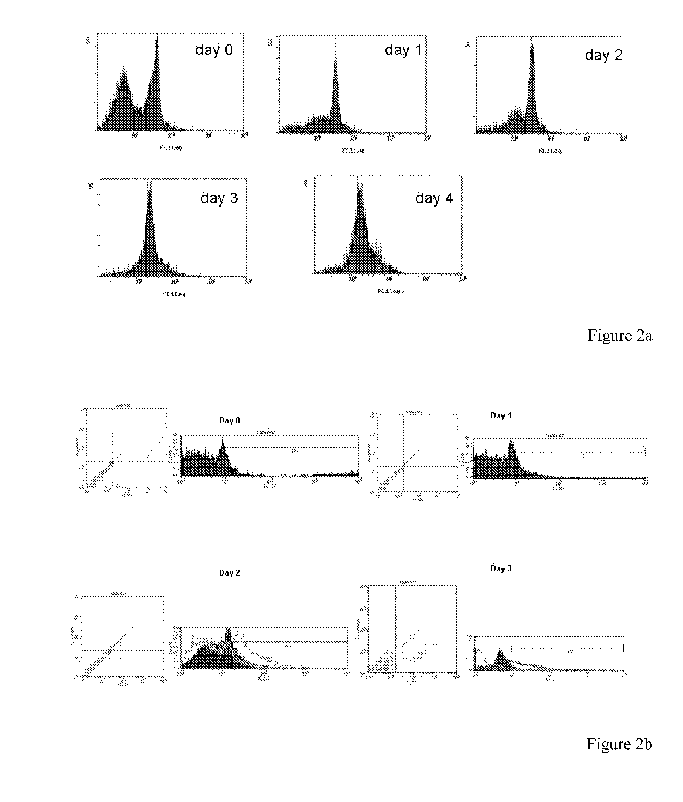

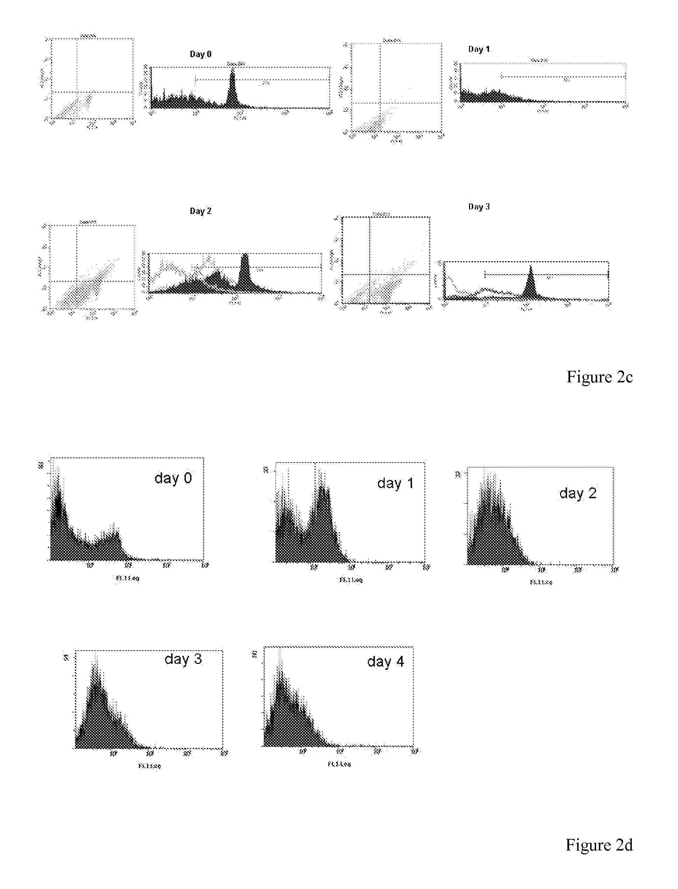

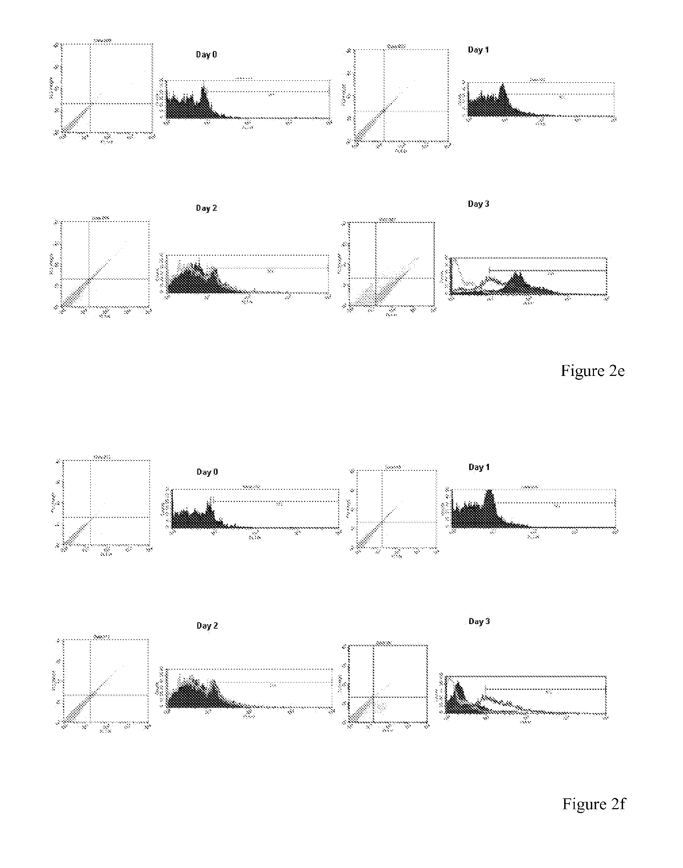

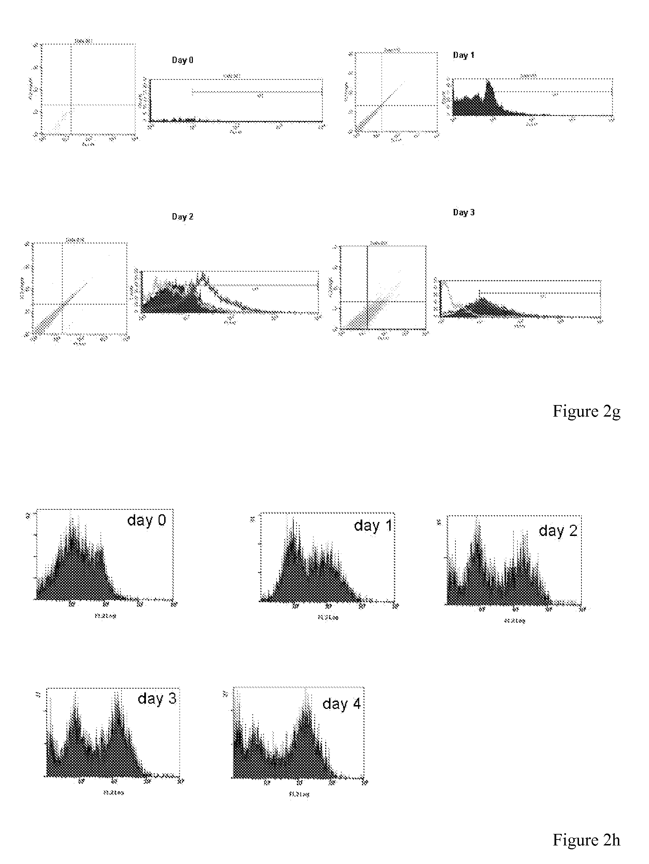

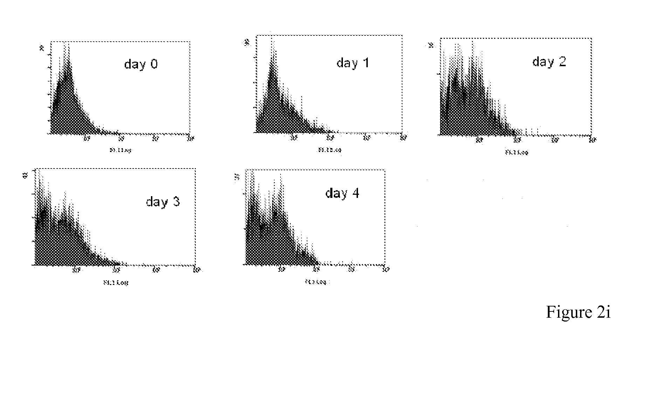

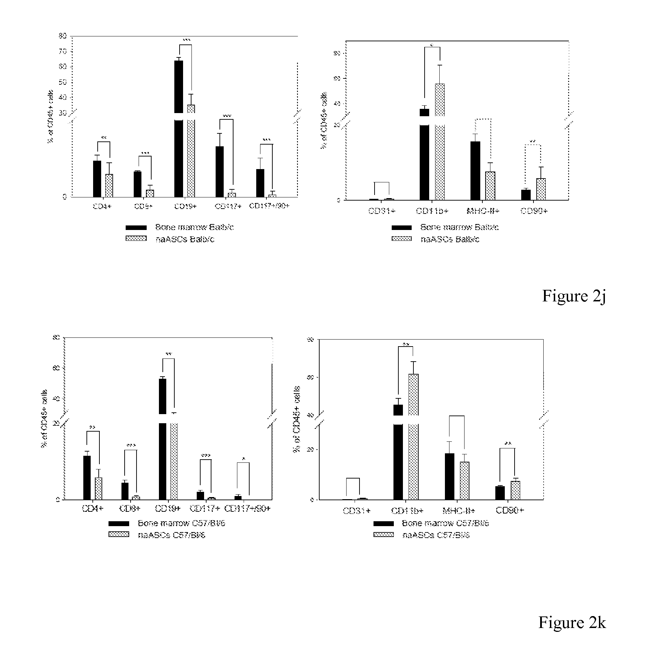

2 x 10⁶ cells were incubated with 2.5µl of conjugated monoclonal antibodies (CD45-PerCP; CD31-FITC; CD90-PE ; CD117-APCCy7; CD11b -APCCy7 (all BD Biosciences, Heidelberg; CD105, CD133, CD44 (all Serotec)). A 25 minutes incubation was followed by two washing steps in PBS/1% FBS (1250rpm, 5min, RT). Finally the pellet was resuspended with 200µl of PBS/3% formaldehyde (Merck, Darmstadt). Data were acquired on a BD FACSCantoII™ Flow Cytometer and analysed using the BD FACSDIVA™ Software (both BD Biosciences). The results of the FACS analysis of the cultured cells are shown in Figure 2.

The results show that the culture system enriches the culture for cells with the following markers: CD44, CD31, CD11b, CD45, CD90, CD105 and CD133, CD45/B 220, F4/80 (see Figures 2a-i). Furthermore, CD4-, CD8-, CD19- and CD117-positive cells were depleted from the CD45 positive subfraction during the culture (Figures 2j and k).

### 4. Analysis of the cells derived from the supernatant for their mesenchymal stem cell properties

### a) Number of CFU-f

After 0, 1, 2 and 3 days of culture as described in Example 1 the supernatant was harvested and 2 x 10⁶ cells of the supernatant per 10 cm petri dish were seeded in 10 ml medium (500 ml DMEM with non-essential amino acids and 4500 mg/l glucose, 5 ml Glutamax, 5 ml Pen/Strep, 5 ml pyruvate and 70 ml fetal calf serum (final cone. 12% v/v)). On the 5^{th} day the medium was changed. From the 5^{th} day on, the medium was changed twice weekly (usually Monday and Thursday). The colonies were inspected daily. When ready, i.e. large and calcified, usually between 12 and 18 days, the cultures were stopped by rinsing them once with PBS, fixed with ethanol for about 5 min and then washed with tap water. The fibroblastic colonies (CFU-f) formed were stained successively for alkaline phosphatase, collagen, calcium and with methylene blue. The plates were always dried, photographed and destained before the next staining step. For methylen blue staining the cells were stained using 1 mg/ml methylene blue in 10 mM borate buffer (prepared from a 1 M borate buffer concentrate adjusted to pH 8.8 with 0.2 N sodium hydroxide) and counted (see Figure 3a). For the calculation of the colony numbers the photographs were loaded into the Photoshop software and adjusted before the analysis of the colony numbers using the method described in Dobson et al. (1999) Tissue Int. 65(2): 166-172. Each CFU-f corresponds to one mesenchymal stem cell. Furthermore, the number of cells in the supernatant of the culture was determined on different days of culture by staining the cells with Trypan blue and counting them in a Neubauer chamber. The result is shown in Figure 3b.

### b) Differentiation of the cells

CFU-f derived from the supernatant of the cell culture on day 4 as described above in a) were stained for different differentiation markers which indicate differentiation of the cells to mesenchymal cells. Colonies staining positive for alkaline phosphatase, calcium and/or collagen were considered as osteogenic differentiated cell, showing the potential of the stem cells of the present invention to differentiate.

For alkaline phosphatase staining 5 ml of APase buffer (20 mM Tris, pH 8,5; 50 µg/ml naphthol phosphate BI; 1 mg/ml fast red) were added to each petri dish and the dishes were shaken at room temperature for 30 minutes. Afterwards, the dishes were washed with tap water and allowed to dry before evaluating the number of colonies which stained positive for alkaline phosphatase. The dishes were destained by shaking them overnight with ethanol and washing with tap water, before they were used for calcium staining.

For calcium staining 5 ml calcium stain (1 mg/ml Alizarin red in distilled water adjusted to pH 5.5 with ammonium hydroxide) were added to each 10 ml petri dish which was previously fixed, destained and washed with tap water, and shaken at room temperature for 30 minutes. Afterwards, the dishes were washed with tap water and allowed to try before evaluating the number of colonies which stained positive for calcium. The dishes were destained with 5% perchloric acid and washed well with tap water, before they were used for collagen staining.

For collagen staining 5 ml collagen stain (1 mg/ml Sirius red in saturated picric acid prepared by leaving an excess of picric acid over distilled water for 18 hours and decanting the saturated picric acid) were added to each petri dish which was previously fixed, destained and washed with tap water, and shaken at room temperature for 18 hours. Afterwards, the dishes were washed with tap water until no more red colouring was eluted before evaluating the number of colonies which stained positive for collagen. The dishes were destained with 0.2 M sodium hydroxide/methanol, before they were used for methylene blue staining (see above). The results of these stainings are shown in Figure 3c.

These results show that the process of the present invention produces mesenchymal stem cells over a longer period of time which stem cells are able to differentiate like mesenchymal stem cells isolated from bone marrow.

### 5. Stem cell transplantation

After 4 days of culture according to Example 1, the cells were washed three times with normal saline (0.9% NaCl) and the cell concentration was adjusted to 2x10⁶ cells per 150/µl of 0.9% Nacl for injection. The TTG mice described in Example 1 were irradiated with the X-Ray apparatus (D3225, Orthovoltage, Gulmay Medical) with an irradiation dose of 8 Gy which was previously determined to be the lethal irradiation dose for the mice. Within seven hours after the irradiation procedure 2 x 10⁶ - 5 x 10⁶ cells in 150µl of 0.9% NaCl were used for intravenous injection into the lateral vein of recipient mice. Engraftment of cells was investigated weekly for 8 weeks.

### 6. Analysis of peripheral blood of recipient mice

Before and after the transplantation procedure, blood of recipient mice was analyzed. At particular time points, blood (150µl) was taken from the retro orbital vein of each mouse under ether anesthesia and collected in heparinized capillaries (Greiner Biochemica, Flacht).

The hemoglobin concentration in the blood was determined using an Animal Blood Counter (SCIL, Viernheim, Germany), which had been calibrated for mouse blood.

For flow cytometric analysis 100µl of blood cells were incubated with 2.5µl of conjugated monoclonal antibodies according to samples (murine CD4 - PE; MHC-I (H-2D[b]) - PE; MHC-I (H-2K[d]) - PE, [BD Biosciences, Heidelberg], murine CD3 - FITC, murine CD8 - APC, human CD4- PE [Beckman Coulter, Krefeld], human HLA-DR3 - FITC [Immunotools, Friesoythe]). 25 minutes incubation was followed by erythrocyte lysing according to manufacturer's instructions (BD FACS Lysing Solution [BD Biosciences]). By adding of PBS/1% FBS samples were washed twice (1250rpm, 5min, RT). Finally, the pellet was resuspended in 200µl of PBS/3% formaldehyde (Merck, Darmstadt). Data were acquired on a BD FACSCantoII ™ Flow Cytometer and analyzed using the BD FACSDIVA™ Software (both BD Biosciences).

### 7. Histological analysis

Liver, bones and gut of irradiated transgenic mice were analyzed histologically. All organs of dying mice were prepared immediately after death of the mice, organs from all other mice at the end of the experiment. The organs were then transferred to formalin (4% w/v, Merck, Darmstadt) for hematoxylin-eosin (HE) and Kaoline-Aniline-Orange G (KAO) staining. Until the further handling, the formalin boxes were kept under dark conditions to prevent formalin precipitation. Bones were incubated in a beaker glass filled with Osteosoft^{®} for at least 7 days at room temperature. All samples were flushed with tap water for 2 hours and then submerged in alcohol series from 70% till 100% for 9 h. The series was concluded by a final incubation with isopropanol for one hour and an overnight incubation with methylbenzoate. After that, the organs were put in a plastic cassette (Tek^{®} histo cassettes) and embedded in paraffin for three days. Paraffin blocks were sliced on a microtome (Leica, Germany) and transferred onto a polylysine slide to dry overnight at 37 °C. HE and KOA staining was done as described previously. The slides were incubated with xylene for 5 min twice at room temperature and passed through a descending alcohol series (100% w/v - 50% w/v) and finally transferred to ddH₂O at room temperature. Object slides were placed in Mayer's hemalaun solution for 5 min and washed with tap water for 10 min to reach a blue staining. After incubation with 1% w/v Eosin Y the slides were passed through an ascending (70%-100% w/v) alcohol series and finally covered with Entellan (Merck, Darmstadt, Germany). The object slides were analyzed under the microscope at room temperature (Nikon, Eclipse TE2000-E. 20x, objective lenses Plan Fluor 20x/0.45 Ph1 DM ∞/0-2 WD 7.4 Histo, Software Nikon, LuciaG 5.00). Osseous containing substances are stained with KAO according to Halmi-Konecny (Chroma Werksschrift. Ausgewählte Färbemethoden für Botanik, Parasitologie, Zoologie Chroma-Ges. Schmid & Co., Stuttgart-Untertürkheim, 1962, page 57).

### 8. Immunohistology

Organs of mice were put in a stainless steel beaker with 2-methylbutane in liquid nitrogen for 15 min and stored at -80 °C until the sectioning process. Sectioning was done using a Cryostat (Leica Biosystems, Nussloch, Germany) and objects were transferred onto a Superfrost slide (Thermo Scientific, Braunschweig, Germany) and stored immediately at -80 ° until the immunohistological analysis was performed. The object slides were incubated with 0.3% w/v H₂O₂, dissolved in PBS (10 x PBS: 140mM NaCl, 2.7mM KCl, 10mM Na₂HPO₄, 1.8mM KH₂PO₄, pH 7.4) for 10 min in a wet chamber and then washed with PBS for three times. Organs were treated with 10% FBS (Gibco/Invitrogen, Auckland, New Zealand) w/v (dissolved in PBS) for 60 min at room temperature, than shortly washed with PBS, incubated with avidin solution (Dako North America, Carpinteria, USA) for 10 min and finally washed with PBS. The preparations were incubated with biotin solution (Dako North America, Carpinteria, USA) for 10 min and finally washed with PBS and incubated with the primary antibody (Rat Anti-Mouse CD4 IgG2aκ, Clone: RM4-5 (BD Biosciences, San Diego, USA) or isotype control Rat polyclonal Ig (BD Biosciences, San Diego, USA), diluted 1:100, for 1 h at room temperature. Afterwards slides are covered with secondary antibody (Biotin-conjugated Goat Anti-Rat IgG2aκ (BD Biosciences, San Diego, USA), diluted 1:100, for 30 min at room temperature and washed with PBS for three times. The object slides were covered with the ready for use Streptavidin-Horseradish Peroxidase (BD Biosciences, San Diego, USA) for 30 min and incubated with DAB dilution (BD Biosciences, San Diego, USA) for 5 min until an obvious intensity of colour is achieved and then washed with ddH₂O for three times. The samples were covered with Mayer's hemalaun solution (Merck, Darmstadt, Germany) for a maximum incubation time of 1 min and then washed with tap water for 10 min to visualize blue staining. The object slides passed through the ascending alcohol series (40%-100% w/v) were incubated with xylene for 5 min and finally covered with Entellan (Merck, Darmstadt, Germany). Slides were analyzed under the microscope (Zeiss, Axio, Imager A1, objectives lenses 20x EX Plan-Neofluar, Axiocam MRc5 Zeiss, AxioVision Release 4.6.3).

### 9. RNA isolation and transcription, PCR for murine CD4

The tissue samples were dissected out as fast as possible and preserved in RNA*later* (Ambion Inc., Austin TX, USA) until RNA extraction. Total RNA from animal tissues was isolated by using the RNeasy^{®} Mini Kit (QIAGEN) following the manufacturer's protocol. The RNA pellet was dissolved in RNase-free H₂O. The concentration of the RNA was assessed with the NanoDrop^{®} Spectrophotometer (NanoDrop Technologies, Wilmington, DE, USA) according to the manufacturer's instructions. Purified total RNA was reverse transcribed into cDNA by incubation with Moloney Murine Leukemia Virus reverse transcriptase (RevertAid H Minus M-MuLV, 200U/µl, Fermentas), Olig(dT) primers (0,5µg/µl, Fermentas), 5x reaction buffer (Fermentas) and 10mM dNTPs for 5min at 70°C, followed by 5min at 37°C and 1h at 42°C. The cDNA was stored at -20°C until analysis. PCR was subsequently performed on a Roche LightCycler (Roche, Grenzach-Wyhlen, Germany) using the QuantiTect SYBR Green PCR Kit (Qiagen) in a 20 µl reaction mix containing 2µl of the cDNA, 2µl primer mix (each 20pmol), 6µl water and QuantiTect SYBR Green Master Mix (with HotStarTaq DNA Polymerase, PCR Buffer, dNTP mix, SYBR Green I, ROX passive reference dye and 5mM MgCl₂). Total RNA input was 100ng in each reaction for all genes. Primer sequences for murine CD4 were 5'-TCC GGG TAC CAG CCT GTT-3' and 5'-AGT GTC ATG CCG AAC CAG-3'. Amplification was performed by real-time PCR using the following conditions: 15min activation and denaturation step at 95°C, followed by repetitive cycles of denaturation at 95°C (15s), annealing at primer-specific annealing temperature (20s) and polymerisation at 72°C (25s). A melting curve of the PCR product was obtained by heating at 70°C for 20s, then increasing to 99°C at a rate of 0.1°C/s while recording SYBR green fluorescence. The assay allows the determination of the murine CD4 molecule in the range from 10⁻² to 10⁸ copies/µl.

### 10. Results of the transplantation experiments

### a) Survival and hematopoiesis

For the determination whether allogeneic and syngeneic naASCs derived from bone marrow could reconstitute hematopoiesis after irradiation, triple transgenic mice received transplants of 2 x 10⁶ naASCs from C57/B1/6 and Balb/c wild-type mice which were enriched by the method of the present invention. As shown in Fig. 5A, the survival of mice that received bone marrow derived naASCs was significantly higher than the control mice which were irradiated with a dose of 8 Gy and 12 Gy, respectively (Figure 5A; 90% versus 0% for syngeneic naASCs, P <.001 after approximately 20 days and 62.5% versus 0% for allogeneic naASCs, P <.01 after approximately 25 days).

The weight courses (as percentage of initial weight) of transplanted mice showed a significant benefit of mice transplanted with 2 x 10⁶ cells of allogeneic and syngeneic naASCs compared to 5 x 10⁶ cells of syngeneic bone marrow control from day 46-50 (P<.001) (Figure 5B). At the end of the experiment on day 50, the percentage of weight for groups transplanted with allogeneic naASCs was 108.6%, for syngeneic naASCs 103% compared to 95.93% of the syngeneic bone marrow control group. The transplantation of 2 x 10⁶ syngeneic bone marrow cells did also preserve 100% of mice from death (data not shown), but reconstitution of hematopoiesis was later (Figure 5C and 5D). By treatment of irradiated mice with 2 x 10⁶ of allogeneic bone marrow cells only 2 of 9mice (22%) survive lethal irradiation.

To demonstrate the X-Ray and transplantation effect on hematopoiesis the blood count of all irradiated mice was determined once a week and a model function was created. Modeling procedure was done for Hb in dependence on dose, time after irradiation and transplantation. We were able to develop a valid mathematical function describing the prediction and time point of recovery of hematopoiesis (shown for Hb, Figure 5C-D). Development of Hb course is a representative marker for measuring influences on the recovery of hematopoiesis. The graphs demonstrate validity of the model. It was shown that after transplantation of 2 x 10⁶ allogeneic and syngeneic naASCs the hematopoiesis recovered earlier to normal levels than after transplantation of 2 x 10⁶ allogeneic and syngeneic bone marrow cells (Figure 5C-D). For syngeneic non adherent naASCs a nearly significant time shift k of 6.7 days was found (Figure 5D; 95% confidence interval contain 0). The tendency for earlier reconstitution was also shown for allogeneic naASCs.

### b) Hematopoietic chimerism after transplantation of allogeneic and syngeneic naASCs

Human and murine CD4 molecules could be used to investigate chimerism after transplantation by flow cytometry, quantitative PCR, and immunohistochemistry.

After transplantation of allogeneic Balb/c derived naASCs the MHC-I expression level from Balb/c (H-2K[d]) remained low (3.1±2.6% on day 40) and the recipient MHC-1 molecules from C57/B1/6 (H-2D[b]) were expressed (94.3±5.4% on day 40) (Figure 6A). The murine CD4 stayed knock out (0.28±0.13%) (Figure 7). The recovery of the human CD4 (30.6±5.9%) and HLADR3 (13.3±4.8%) molecules indicates recurrence of recipient hematopoiesis (Figure 8). First occurrence of murine CD4 was on day 40 and persistent to the end of the experiment (data not shown).

After transplantation of syngeneic C57/B1/6 derived naASCs, recurrence of MHC-I from C57/B1/6 (H-2D[b]) molecule was 98.8±0.8% on day 40 (Figure 6B), whereas Balb/c (H-2K[d]) molecules remained low (0.94±0.15%) as estimated. For murine CD4 a hematopoietic chimerism of 5.8±2.4% on day 40 could be observed (Figure 7; P < 0.001) indicating the engraftment of transplanted syngeneic cells. The recurrence of human HLA molecules (2.6±0.28%) and human CD4 (36.5±1.1%) after transplantation indicate the parallel recurrence of recipient hematopoiesis (Figure 8).

The investigation of distribution of MHC-I from Balb/c (H-2K[d]) and of MHC-I molecules from C57/B1/6 (H-2D[b]) shows that the transplantation of 2 x 10⁶ normal bone marrow cells derived from Balb/c leads to an expression of Balb/c (H-2K[d]), murine CD3, murine CD4 and murine CD8 on lymphocytes after engraftment in triple transgenic mice (Figure 6C). However by using the irradiation conditioning therapy only 2 of 9 mice survive and show engraftment of Balb/c bone marrow cells.

### c) Organ recovery before and after transplantation

To evaluate the organ and tissue damage before transplantation the organs of irradiated and transplanted mice were investigated. Mice were irradiated with X ray doses from 8 Gy. Figure 9 shows the histology of the bone marrow cavities, the gut system and the liver of irradiated control and transplanted mice. Before irradiation there are hematopoietic islets with a prevalent form of erythropoiesis. The administration of X Rays with a radiation dose of 8 Gy was lethal. A reduced cellularity (< 30%) with a loss of bone marrow and a replacement of bone marrow cells by adipose cells was found.

The gut system shows lesions of the mucosa (oedematous with an inflammable cellular infiltration and ulcera).

Lymphatic system is clearly hyperplastic and expanded transmural cicatrices occur.

Liver tissue is less sensitive for X-Ray irradiation. A fatty degeneration of the liver tissue and necrosis of liver cells was found. Because of the leukopenia, the cell density and the decomposition of necrotic cells by leukocytes are low. To determine therapeutic and repair effects of transplanted cells organs and tissues of all transplanted animals were examined histologically. After transplantation of allogeneic and syngeneic naASCs and bone marrow the bone marrow cavities showed regeneration with a normal distribution of blood cells. There was a recurrence of islets with prevalent form of erythropoiesis, endothelial cells and reticulum cells. The gut system shows an intact barrier and on organs no signs of tissue destruction were visible.

### d) Murine CD4 expression in gut after transplantation of allogeneic and syngeneic naASCs

Analysis of murine CD4 expression by immunohistochemistry and RNA expression by PCR were chosen as a diagnostic marker for the engraftment of transplanted cells and regeneration of radiation damaged organs. By analyzing gut by immunohistochemistry, it was possible to show that after transplantation of syngeneic naASCs derived from C57/B1/6 the murine CD4 molecule was detectable. In contrast, in case of allogeneic naASCs the murine CD4 was not detected (Figure 10A-H). The PCR results also confirmed that after transplantation of allogeneic naASCs from Balb/c the murine CD4 expression in the recipient remained knocked out compared to syngeneic naASCs derived from C57/B1/6 (Figure 10I).

## Claims

1. Method of enriching stem cells in cell culture, comprising the steps of:
a) culturing a cell mixture comprising stem cells in a suitable cell culture medium in a culture vessel;
b) transferring the supernatant comprising non-adherent cells to a new culture vessel in intervals of 6 to 32 hours; and
c) harvesting the enriched stem cells from the cell culture supernatant.

2. Method according to claim 1, wherein the supernatant is transferred to a new culture vessel at least three times.

3. Method according to any of the preceding claims, wherein the cell culture medium is not exchanged during the transfer of the cells.

4. Method according to any of the preceding claims, wherein the culture vessel is treated for tissue culture.

5. Method according to any of the preceding claims, further comprising the step of seeding the cell mixture comprising the stem cells in a density of 10⁵ to 10⁷ cells per ml in the cell culture medium before culturing the cell mixture comprising the stem cells.

6. Method according to any of the preceding claims, wherein the cell mixture comprising the stem cells is obtained from a suitable stem cell source, preferably from bone marrow.

7. Method according to any of the preceding claims, wherein the stem cells are of mammalian origin, preferably of human origin.

8. Method according to any of the preceding claims, wherein the enriched stem cells are positive for at least one marker selected from the group consisting of CD44, CD31, CD11b, CD45, CD90, CD105 and CD133.

9. Stem cells enriched by a method according to any of claims 1 to 8.

10. Pharmaceutical composition comprising the stem cells according to claim 9.

11. Stem cells according to claim 9 for use as a medicament.

12. Use of the stem cells according to claim 9 in the preparation of a medicament for the reconstitution of hematopoiesis, for the treatment of Alzheimer's disease and/or ageing or for bone healing.

13. Isolated cell population which is positive for the markers CD44, CD31, CD11b, CD45, CD90, CD105 and CD133.

14. Pharmaceutical composition comprising the isolated cell population according to claim 13.

15. Isolated cell population according to claim 13 for use as a medicament.

16. Use of the isolated cell population according to claim 13 in the preparation of a medicament for the reconstitution of hematopoiesis, for the treatment of Alzheimer's disease and/or ageing or for bone healing.
